# EUROPEAN PATENT APPLICATION

(11) **EP 2 684 541 A1**
(43) Date of publication of application: **15.01.2014**
(21) Application number: 13175083.8
(22) Date of filing: 04.07.2013
(51) Int. Cl.: A61F 2/30

(54) **A joint fluid drainage system for the problem of aseptic loosening in the total joint prostheses**

(30) Priority: 11.07.2012 TR 201208089 U
(71) Applicant: Parmaksizoglu, Ahmet Fatih, Istanbul (TR)
(72) Inventor: Parmaksizoglu, Ahmet Fatih, Istanbul (TR)
(74) Representative: Iskender, Ibrahim

(57) **Abstract**

The present invention is intended for providing a solution to the aseptic loosening problem resulting from the utilization, for a certain period of time, of total joint prostheses, lowering the pressure inside the joint capsule and draining the joint fluid comprising particles outside the joint capsule. Thus, it relates to a drainage system comprising a canal outlet starting from the articulation surface. This high pressure joint fluid with particles causing the loosening is drained from the capsule to the outside of the bone by means of the drainage path starting from this canal outlet, continuing in the prosthesis and the bone, and opening to the outer surface of the bone. In addition, the system also comprises a silicone tube mounted to the canal outlet of the prosthesis by using the canal disposed in the bone. The silicone tube is placed inside the fatty porous tissue located between the muscle groups outside the bone and the joint fluid is discharged into this area through this canal system by drainage.

## Description

### TECHNICAL FIELD

The present invention relates to a joint fluid drainage system providing a solution to the aseptic loosening problem resulting from the utilization, for a certain period of time, of total joint prostheses used in the orthopedic surgery for the treatment of joint disorders and serving for lowering the pressure inside the joint capsule.

Total joint prostheses should necessarily comprise two artificial surfaces working by rubbing against each other and made of the same or different materials. The wear between these surfaces and the particles coming off these materials to fall into the environment is inevitable.

There are basically two reasons for aseptic loosening.

The first of these is that the amount of the joint fluid increases due to the irritation caused by the particles resulting from the friction of the artificial surfaces rubbing against each other in line with the movement of the joint prosthesis falling into the joint capsule. Since the joint capsule is a closed environment, the increasing amount of fluid causes the pressure to increase. This pressure can increase up to four times of the blood pressure. With this pressure, the joint fluid enters in between the bone and the prosthesis, or the bone and the cement, and causes inflammation of the bone tissue and formation of a reactive aseptic soft tissue.

Other than the high pressure, the second reason for aseptic loosening is these falling particles. These particles create the irritation formed thereby inside the joint also inside the bone tissue when the high pressure joint fluid starts to leak in between the bone and prosthesis, or the bone and cement, and cause the formation of a reactive soft tissue between the bone and prosthesis, or bone and cement, leading to loosening. A design change lowering the pressure as well as removing the particles from the environment to prevent loosening can eliminate the two most important reasons of aseptic loosening. The present invention relates to a drainage system capable of providing solution to or substantially delaying this aseptic loosening problem and enabling the intracapsular high pressure joint fluid and the particles disposed therein to be removed from the environment.

### STATE OF THE ART

Today, total joint prostheses are widely used in the orthopedic surgery for treatment of joint disorders. In this surgical practice, which has obtained promising results in the recent years, one of the most important problems arising in late stage is the so called aseptic loosening, weakening of the bone-prosthesis or bone-cement connection. When this problem occurs, a new prosthesis should be implanted by removing the loose prosthesis. Intensive studies are carried out all over the world to eliminate said loosening problem, wherein said problem is tried to be resolved by means of material and design changes in the prostheses.

In the literature, the application no TR 2005 05215 titled 'Total joint prosthesis model developed for preventing the aseptic loosening observed in some total joint prostheses used in the orthopedic surgery for treatment of some joint disorders' can be given as an example. Said application is a model provided with development of a total joint prosthesis enabling to prevent the aseptic loosening observed in the prostheses used in the orthopedic surgery for treatment of some joint disorders. Two main prosthesis parts are used for forming a joint surface for each of the articulated bones in the knee and hip prostheses. The loosening problems encountered in the treatments performed with total prosthesis application are overcome with the design change comprising a tunnel disposed in the distally located part of these two main parts, connecting the medullary canal of the bone and the joint cavity to each other at the lower level of the joint cavity and enabling the high pressure joint fluid to be discharged; a tunnel (6) disposed in the prostheses for the hip; and a tunnel (10) disposed in the prostheses for the knee.

In a closed space, absorption of the fluid can be a problem; in addition, since the bone is a closed volume without any possibility of expansion, said volume is not a suitable place for discharging the fluid.

Furthermore, again in the same application, the discharge outlet disposed inside the joint is located in the neck of the prosthesis. In the postoperative recovery period, it is likely that said discharge outlet is blocked by the scar tissue.

Consequently, the joint cavity of the prostheses used in the current prosthesis surgery is a closed structure, wherein no design for discharging the joint fluid stimulated by the resulting particles outside the joint capsule, thus lowering the pressure of the fluid inside the joint, is provided. Thus, new configurations that will eliminate the aforementioned drawbacks and offer solutions to the existing systems are needed.

### OBJECTS OF THE INVENTION

The present invention relates to a joint fluid drainage system, which meets the aforementioned requirements, eliminates all the drawbacks and brings additional advantages.

The object of the present invention is to delay or prevent the aseptic loosening problem by allowing for discharging the joint fluid disposed inside the capsule in the prostheses outside the joint capsule.

Another object of the present invention is to completely prevent the possibility of the discharge outlet being blocked by the scar tissue during the postoperative recovery period by moving the discharge outlet to the articulation surface of the prosthesis. With this new design, blockage of the discharge outlet is not possible.

The present invention, in order to achieve the aforementioned objects, relates to a joint fluid drainage system comprising a canal outlet, wherein it comprises a canal associating the bone canal extending from the outer surface of the bone towards the bone with the articulation surface of the prosthesis.

The present invention, in the preferred embodiment thereof, comprises a canal drilled in said prosthesis for forming a continuous canal path along with said bone canal.

In the preferred embodiment of the present invention said drainage tube is made of silicone.

In the preferred embodiment of the present invention, the connection of said drainage tube with the bone canal is at the screwing point.

In the preferred embodiment of the present invention, the drainage tube disposed inside the bone canal and connected with a screw to the prosthesis is made of silicone, wherein it drains the fluid inside the joint capsule to the outer surface of the bone, to the fatty porous tissue between the muscles.

In the preferred embodiment of the present invention, said canal outlet opens to the articulation surface of the prosthesis for preventing the end of said canal outlet disposed inside the joint from being covered or blocked by the scar tissue. In this moving area, the development of scar tissue is not possible.

The structural and the characteristic features and all advantages of the present invention will be understood more clearly with the following figures and the detailed description written by referring to said figures; therefore, the evaluation needs to be done by taking said figures and detailed description into consideration.

### BRIEF DESCRIPTION OF THE FIGURES

Embodiment of the present invention and advantages thereof with the additional components must be considered together with the figures explained below in order to be fully understood.
**Figure 1****:** A view of the drainage canal femoral component for the hip joint.
**Figure 2****:** A view of the drainage canal tibial component for the knee joint.
**Figure 3****:** A view of the guide to be used in forming bone canal in the hip joint and implementation method thereof.
**Figure 4****:** A view of the guide to be used in forming bone canal in the knee joint and implementation method thereof.
**Figure 5a****:** A cross-sectional view of the grooves disposed on the prosthesis surface of the knee prosthesis (on the plastic insert surface) where the groove-canal outlet-canal relationship is shown.
**Figure 5b****:** A top view of the plastic insert.
**Figure 5c****:** A side view of the plastic insert.
**Figure 5d****:** A view of the silicone drainage tubes with threaded end discharging the high pressure and particle rich intrajoint fluid and screwed to the components.

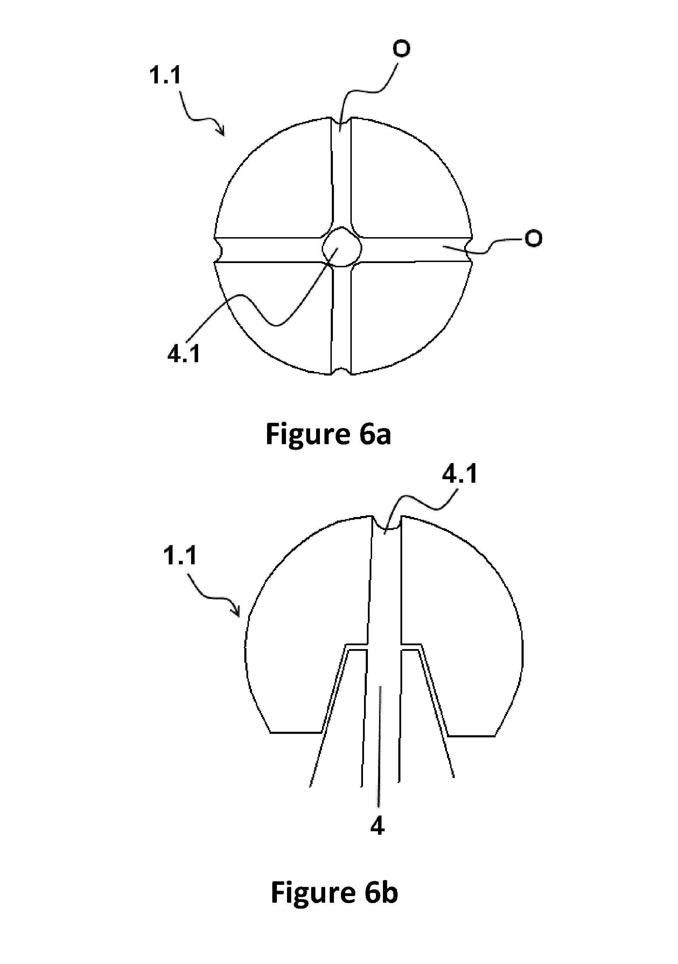

**Figure 6a****:** A top view of the prosthesis head of the hip prosthesis. (Prosthesis head and grooves and groove-canal outlet-canal relationship)
**Figure 6b****:** A cross-sectional view of the prosthesis head of the hip prosthesis.

**REFERENCE NUMBERS OF THE PARTS**

| | | | |
|---|---|---|---|
| **1.** | Femoral or tibial component (prosthesis) | **5.** | Silicone drainage tube |
| | | **5.1.** | Silicone drainage tube screwing point |
| **1.1.** | Head portion of the prosthesis | **6.** | Guide used for drilling canal in the bone |
| **2.** | Femur or tibia (bone) | **S:** | Screw |
| **2.1.** | Canal drilled in the bone | **D:** | Drill bit |
| **3.** | Joint capsule | **x:** | Inner |
| **3.1.** | Intracapsular cavity | **y:** | Outer |
| **4.** | Drainage canal inside the prosthesis | **G:** | Groove |
| **4.1.** | Drainage canal outlet | **I**: | Insert |

Scaling of drawings is not absolutely required and details which are not needed for understanding the present invention could have been neglected. Furthermore the elements which are at least substantially identical or have at least substantially identical functions are indicated with the same number.

### DETAILED DESCRIPTION OF THE INVENTION

In this detailed description, preferred embodiments of the joint fluid drainage system of the joint prostheses (1) according to the present invention are described only for a better understanding of the subject without constituting any restrictive effect.

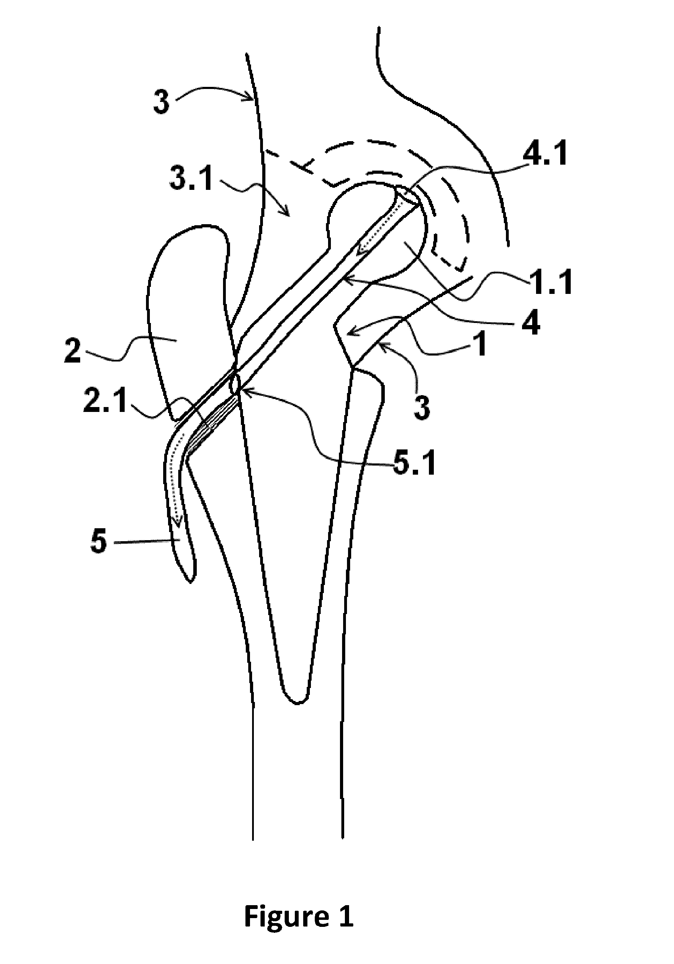

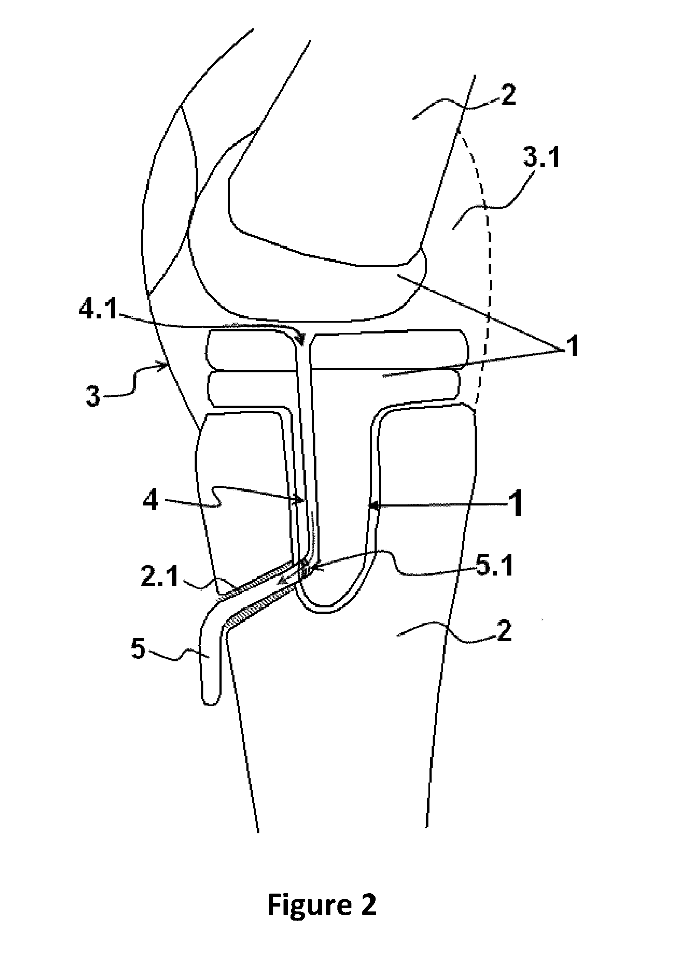

In Figure 1 and Figure 2, the femoral and tibial components (1) provided with a canal (4) connecting the joint cavity (3.1) to the outer portion of the capsule (3), to the outer surface of the bone (2) implanted with the prosthesis (1) by means of a canal (2.1) drilled in the bone starting from the articulation surface, i.e. from the head (1.1) portion of the prosthesis and advancing through the prosthesis (1) are shown.

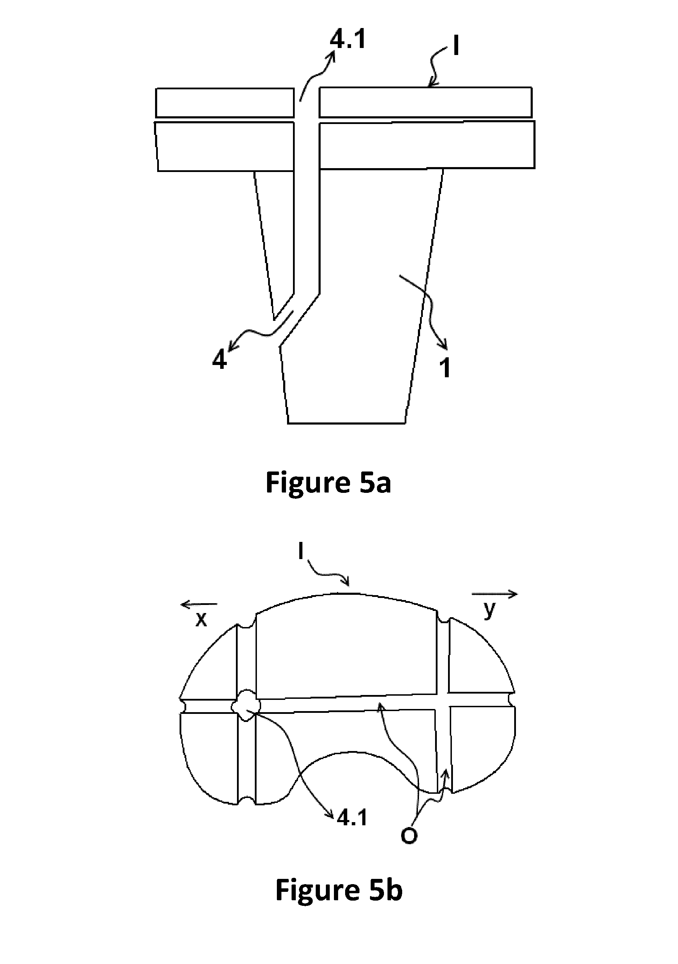

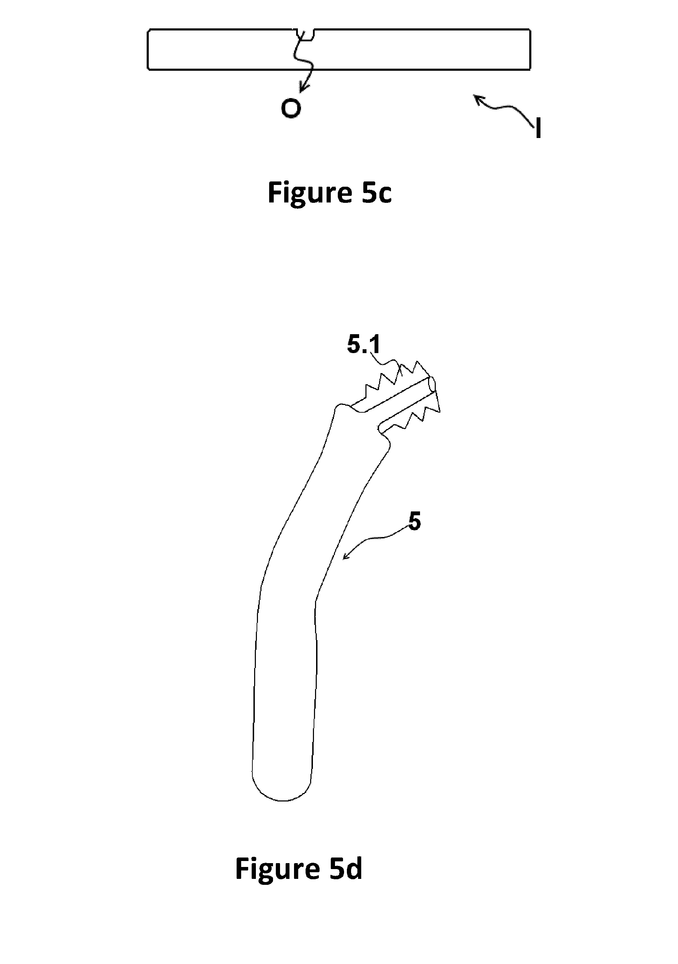

In Figure 5d, the silicone drainage tube (5), which is a continuation of the joint fluid drainage canal (4) mentioned in Figure 1 and Figure 2, is shown. Said silicone drainage tube (5) is connected through the outer surface of the bone (2) where it is placed, to the prosthesis (1) inside the bone by means of a canal (2.1) drilled in the bone. Thus, the environment inside the joint is completely connected with outside of the bone (2). Said silicone drainage tube (5) is inserted under the fascia, into the fatty porous tissue.

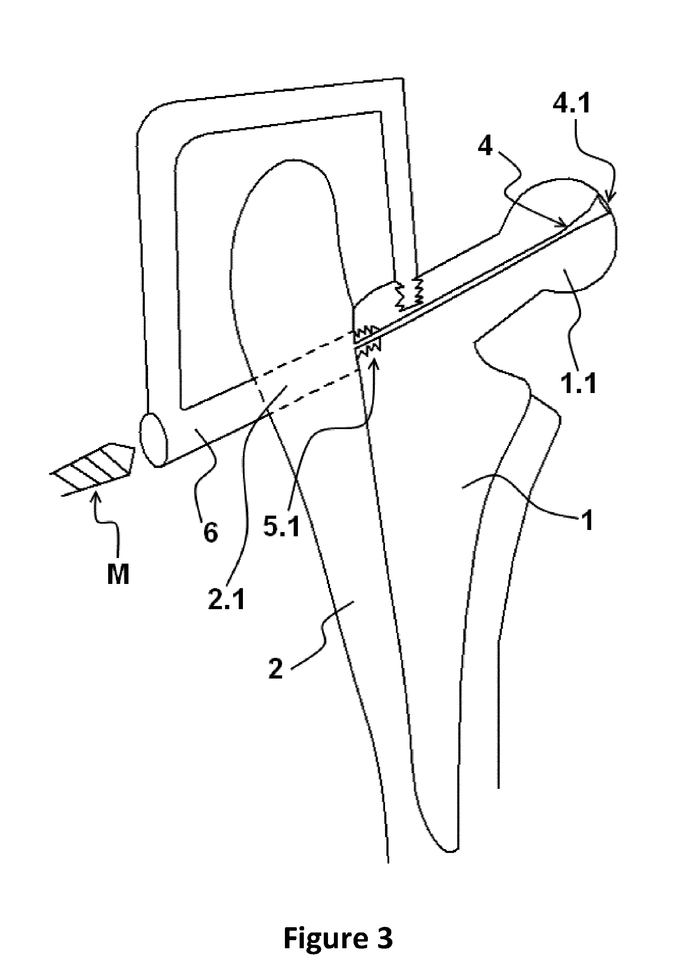

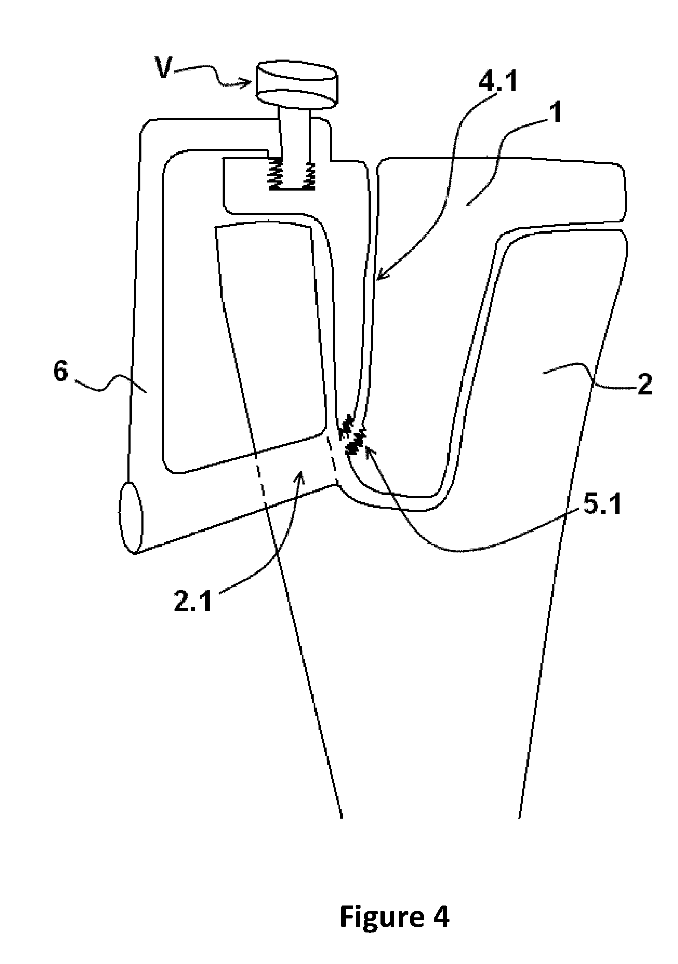

In Figure 3 and Figure 4, implementation method, by means of a guide (6), of the canal (2.1) required to be drilled in the bone (2) for connection of the silicone drainage tube (5) with the prosthesis (1) disposed inside the bone (2) is illustrated. After driving of the prosthesis (1) into the bone (2) is completed, a guide (6) is temporarily fixed by means of a screw (S) to the prosthesis (1) provided with the drainage canal (4) therein. By means of this guide (6), a guidance from the outer surface of the bone (2) for drilling a canal (2.1) inside the bone (2) corresponding to the outlet of the drainage canal (4) disposed on the prosthesis (1) provided in the inner part is obtained. With the guidance of this guide (6), a canal (2.1) is drilled in the bone (2) reaching the prosthesis (1) from the outer surface of the bone (2) by using a drill bit (D). By means of using this canal (2.1), the silicone drainage tube (5) can be screwed to the prosthesis (1) inside the bone (2) through the screwing point (5.1) by using an application tool.

For the high pressure joint fluid to reach the discharge canal outlet (4.1);
**a- In the hip joint,** a '+' shaped groove (G) having a depth of 2 mm and a width of 4 mm is disposed on the articulation surface of the head of the prosthesis (1). The environment required for fluid drainage is provided and the blockage of the canal outlet (4.1) by the opposite joint surfaces sitting firmly against each other is prevented thanks to said groove (G) system.
**b- In the knee joint,** grooves (G) forming an interconnected '+' shape on the two surfaces (inner-outer (x-y)), having a depth of 2 mm and a width of 4 mm are disposed on the polyethylene insert (I) placed between the two metal surfaces bonded to the tibia and femur (2). The center of the inner (x) '+' is drilled on the drainage canal (4) disposed inside the prosthesis (Figure 5b).

Said silicone drainage tube (5) discharges the high pressure joint fluid with particles to the fatty porous tissue outside the bone having a lower pressure. The fluid leaking in between the bone and prosthesis, or bone and cement, due to the high pressure drains into the fatty porous tissue in a much easier manner.

## Claims

1. A joint fluid drainage system providing a solution to the aseptic loosening problem resulting from the utilization, for a certain period of time, of total joint prostheses and comprising a canal outlet (4.1) for lowering the pressure inside the joint capsule (3) and removing the particles therefrom, **characterized in that** it comprises a canal (4) associating the canal (2.1) drilled through the outer surface of the bone (2) with the articulation surface of the prosthesis and providing drainage.

2. The joint fluid drainage system according to claim 1, **characterized in that** it comprises a drainage tube (5) connected to the head of the canal (2.1) drilled in the bone (2).

3. The joint fluid drainage system according to claim 1, **characterized in that** it comprises a canal (4) drilled in said prosthesis (1) for forming a continuous canal path along with said bone canal (2.1).

4. The joint fluid drainage system according to claim 2, **characterized in that** said drainage tube (5) is made of silicone.

5. The joint fluid drainage system according to claim 1, **characterized in that** the connection of said drainage tube (5) with said bone canal (2.1) is at the screwing point (5.1).

6. The joint fluid drainage system according to claim 1, **characterized in that** said canal outlet (4.1) is disposed on the articulation surfaces of the prosthesis for preventing the end of said canal outlet (4.1) disposed inside the joint from being covered or blocked by the scar tissue.
